# EUROPEAN PATENT SPECIFICATION

(11) **EP 1 562 492 B1**
(45) Date of publication and mention of the grant of the patent: **03.01.2007**
(21) Application number: 03789772.5
(22) Date of filing: 17.11.2003
(51) Int. Cl.: A61B 17/122, A61B 17/28, A61B 8/06

(54) **APPARATUS FOR THE DETECTION AND OCCLUSION OF BLOOD VESSELS**
VORRICHTUNG ZUR DETEKTION UND OKKLUSION VON BLUTGEFÄSSEN
APPAREIL DE DETECTION ET D'OCCLUSION DE VAISSEAUX SANGUINS

(30) Priority: 19.11.2002 US 300115
(43) Date of publication of application: 17.08.2005
(73) Proprietor: Vascular Control Systems, Inc., San Juan Capistrano, CA 92675 (US)
(72) Inventor: BURBANK, Fred, H., Laguna Niguel, CA 92677 (US); JONES, Michael, L., San Clemente, CA 92673 (US); SERRA, R., J., Irvine, CA 92612 (US); ALTIERI, Greig, E., Laguna Beach, CA 92651 (US); UYENO, Jill, Mission Viejo, CA 92691 (US); WONG, Yu-Tung, Irvine, CA 92614 (US); WERNETH, Randy, Poway, CA 92064 (US)
(74) Representative: Barz, Peter
(86) International application number: PCT/US2003/036553
(87) International publication number: WO 2004/045426

(56) References cited:
- WO-A-99/11179
- GB-A- 2 302 025
- US-A- 3 777 740
- US-A- 5 598 841

## Description

### FIELD OF THE INVENTION

The invention relates generally to the field of treatment of diseases and conditions by the regulation of blood flow in blood vessels. In particular, the invention is directed to the treatment of uterine conditions by detecting and regulating blood flow thereto.

### BACKGROUND OF THE INVENTION

Hysterectomy (surgical removal of the uterus) is performed on approximately 600,000 women annually in the United States. Hysterectomy is often the therapeutic choice for the treatment of uterine cancer, adenomyosis, menorrhagia, prolapse, dysfunctional uterine bleeding (abnormal menstrual bleeding that has no discrete anatomic explanation such as a tumor or growth), and muscular tumors of the uterus, known as leimyoma or uterine fibroids.

However, hysterectomy is a drastic treatment, having many undesirable characteristics. Thus, any method which can approximate the therapeutic result of a hysterectomy without removing the uterus would be a significant improvement in this field. Newer treatment methods have been developed for some diseases which may spare these women a hysterectomy.

In 1995, it was demonstrated that uterine fibroids could be treated without hysterectomy using a non-surgical therapy, specifically comprising bilateral intraluminal occlusion of the uterine arteries (Ravina et al., "Arterial Embolization to Treat Uterine Myomata", Lancet Sept. 9, 1995; Vol. 346; pp. 671-672). This technique is known as "uterine artery embolization". In this technique, uterine arteries are accessed via a transvascular route from a common femoral artery into the left and right uterine arteries.

The uterus has a dual (or redundant) blood supply, the primary blood supply being from the bilateral uterine arteries, and the secondary blood supply from the bilateral ovarian arteries. Consequently, when both uterine arteries are occluded, i.e. bilateral vessel occlusion, the uterus and the fibroids contained within the uterus are both deprived of their blood supply. However, as demonstrated by Ravina et al., the effect on the fibroid is greater than the effect on the uterus. In most instances, the fibroid withers and ceases to cause clinical symptoms.

However, many physicians do not possess the skill or equipment necessary to perform catheter-based uterine artery embolization under radiologic direction. Accordingly, only thousands of uterine artery embolizations have been performed, worldwide, over the past three years, whereas hundreds of thousands of hysterectomies have been performed each year for uterine fibroids which are symptomatic.

What is needed, therefore, are devices to detect blood vessels and blood flow in blood vessels, and devices to occlude blood flow in blood vessels such as the uterine arteries that can be used by physicians of ordinary skill in a simple medical setting or environment. WO99/11179 discloses a typical clamp for temporarily occluding a blood vessel according to the preamble of claim 1.

### SUMMARY OF THE INVENTION

The invention is directed to non-invasive devices, and systems for extravascularly detecting blood flow in a blood vessel, and for occluding an a blood vessel effective to reduce or abolish blood flow in it. The non-invasive devices, and systems embodying features of the invention are configured to be non-surgically applied externally of a blood vessel which they occlude, and are preferably applied at least in part extracorporeally. The occlusion is temporary, and may be partial or complete. An exemplary method of occluding a blood vessel comprises , clamping the blood vessel effective to compress it so that blood flow through the vessel is reduced, or is abolished. Such clamping of a blood vessel may be direct or may be indirect. Preferably, clamping of a blood vessel effective to compress it is accomplished by applying a non-invasive blood vessel occlusion device to tissue near to a blood vessel (e.g., onto tissue surrounding the vessel). A blood vessel occlusion device may also be applied directly onto a blood vessel effective to compress the blood vessel.

In one embodiment of the invention, a non-invasive blood vessel occluding device (such as a clamp with a sensor) may be applied to a portion of a vaginal wall to detect and/or locate, and to occlude the uterine arteries. A vaginal clamp embodying features of the invention may used to sense the location of a uterine artery adjacent a vaginal wall, and may be used to compress and occlude a uterine artery adjacent a vaginal wall. The vaginal wall may be distended by an occlusion device so as to more closely approach a uterine artery; such an approach may aided by applying pressure or force to the uterus (e.g., by pulling on the uterine cervix). A uterine cervix may be grasped or pulled by any suitable device or implement, including forceps, suction devices, and other instruments, such as a tenaculum.

A non-invasive blood vessel occluding device embodying features of the invention may be a non-invasive intravaginal uterine artery occlusion device, comprising a pair of pressure-applying members having opposed tissue-contacting surfaces on distal portions thereof; at least one supporting shaft extending from a proximal extremity of at least one of the pressure-applying members which is configured to adjust the distance between the opposed tissue-contacting surfaces of the pressure-applying members; and at least one blood flow sensing sensor on one of the opposed tissue-contacting surfaces. An embodiment of a non-invasive blood vessel occlusion device embodying features of the invention may have, for example, a handle, a clamping member configured to apply pressure or force to body tissue, and a sensor for locating a blood vessel.

A pressure-applying member, such as a clamping member, may be, e.g., a jaw or jaws configured to engage a blood vessel or to engage tissue adjacent a blood vessel. A supporting shaft, such as a handle, is preferably configured for manipulating the jaw or jaws. In some embodiments of devices having features of the invention, a pressure-applying member may be attached to a connecting portion that is configured so that a jaw may be placed within a vagina while a handle remains outside a patient's body and available for use by an operator.

A sensor for locating a blood vessel may sense sound, pulsation, blood flow or other indicator related to a blood vessel. Thus, a sensor for locating a blood vessel may be a blood flow sensor, a sound sensor, a pressure sensor, a strain sensor, a stress sensor, a chemical sensor, an electromagnetic radiation sensor, or other sensor, and may be a combination of such sensors. A sound sensor may be an ultrasound sensor, including a Doppler ultrasound sensor. The sensor for locating a blood vessel, including a sensor for measuring blood flow, is preferably disposed in or on a pressure-applying member, and is preferably mounted to the face of a tissue-contacting surface, such as the face of a jaw of a clamp. A sensor is preferably oriented perpendicularly to the clamp face, although in embodiments of devices having features of the invention a sensor may assume other orientations.

A system embodying features of the invention may include an blood vessel occluding device having a pair of pressure-applying members configured to apply pressure or force to body tissue, at least one supporting shaft, a sensor for locating a blood vessel, and a sensor controller which may include an energy source. A system may further include a device for grasping a portion of a patient's body, such as a device for grasping a uterine cervix.

A sensor controller may be configured to aid in detecting a location of a blood vessel, by, e.g., providing a signal related to the output of a sensor that may be readily used by an operator. A sensor controller may include an energy source configured to provide energy for operating a sensor for sensing a location of a blood vessel, such as ultrasound energy, electrical energy, or electromagnetic energy. The energy may be directly provided by the energy source or may be provided by the sensor with the aid of the energy source. Ultrasound energy useful for sensing a location of a blood vessel or of blood flow in a blood vessel may have a frequency of less than about 20 MegaHertz (MHz), such as between about 5 MHz and about 19 MHz, preferably between about 6 MHz and about 10 MHz, more preferably a frequency of about 8 MHz. Electromagnetic energy useful for sensing a location of a blood vessel or of blood flow in a blood vessel may have a wavelength of between about 500 nanometers (nm) and about 2000 nm, preferably between about 700 nm and about 1000 nm.

An exemplary method for occluding a blood vessel may include locating a blood vessel with a sensor and compressing a portion of the blood vessel with a non-invasive blood vessel occluding device which includes the sensor. A method of occluding a uterine artery of a patient may include locating a uterine artery with a sensor and compressing a portion of the uterine artery with a non-invasive blood vessel occluding device which includes the sensor. Compressing a portion of a uterine artery may include applying pressure or force to a vaginal wall. In addition, the exemplary methods for occluding a uterine artery include applying tension to a uterus and applying pressure or force to a vaginal wall, and include engaging a uterine cervix with a grasping implement (e.g., by pulling on the uterine cervix) while applying force or pressure to a vaginal wall to occlude a uterine artery.

The non-invasive devices, and systems embodying features of the invention allow the non-surgical location and occlusion of blood vessels, providing therapeutic temporary, partial or complete, reduction or abolition of blood flow in the located and occluded blood vessels. Use of the devices, systems and methods of the present invention thus allow the occlusion of a blood vessel without the puncture of bodily tissue, and without the need for radiographic equipment or for skill in the use of radiographic techniques. The devices are simpler and more readily used and removed than other methods and devices, and provide improved treatments for serious conditions and diseases, including uterine fibroids, dysfunctional uterine bleeding (DUB), adenomyosis, post-partum hemorrhage, and other uterine disorders. The devices, and systems embodying features of the invention thus provide tools for effective treatment of diseases and conditions that otherwise require invasive and irreversible treatments such as removal of a uterus.

### BRIEF DESCRIPTION OF THE DRAWINGS

Figure 1 is a plan view of a system embodying features of the invention including a vaginal clamp embodying features of the invention disposed in an open configuration.

Figure 2 is a fragmentary sectional view of a distal portion of a clamping device embodying features of the invention in a closed configuration.

Figure 3 is a perspective view of a jaw portion of a vaginal clamp embodying features of the invention disposed in an open configuration.

Figure 4 is a transverse cross-sectional view of a jaw portion of the clamping device of Figure 3 taken at line 4-4.

Figure 5 is schematic diagram of a reproductive system of a human female including major blood vessels providing blood flow to the uterus.

Figure 6 is a schematic diagram illustrating the use of a vaginal clamp embodying features of the invention in the occlusion a uterine artery of a female human patient.

### DETAILED DESCRIPTION OF THE PREFERRED EMBODIMENTS

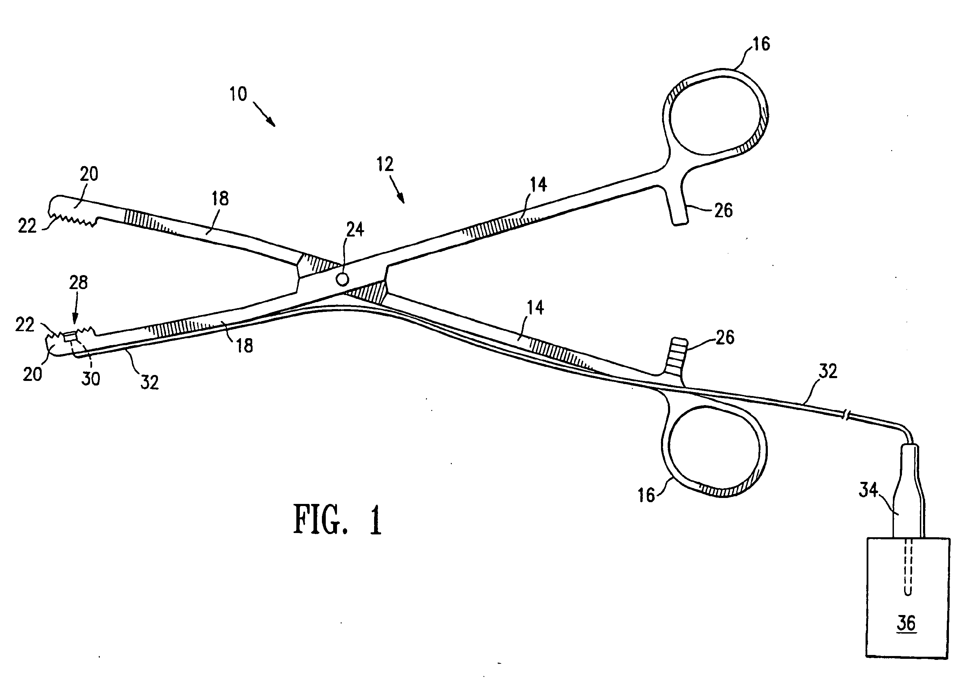

Figures 1-4 show a non-invasive blood vessel-occluding system 10 embodying features of the invention. The system 10 includes a clamping component 12, including handles 14, having finger holes 16, and pressure-applying members 18 with jaws 20 on their distal ends. Jaws 20 have serrated tissue-contacting surfaces 22 configured to engage and hold onto tissue when jaws 20 are pressed into a patient's body tissue. Pressure-applying members 18 are pivotally connected with each other at pivot point 24; handles 14 (which act as supporting shafts for device 12) are also integral with the pressure-applying members 18 and are pivotally connected with each other at pivot point 24. Squeezing handles 14 together, preferably by fingers of an operator's hand engaged with finger holes 16, is effective to cause tissue-contacting surfaces 22 to approach one another as pressure-applying members 18. Such motion may provide mechanical advantage where the lengths of pressure-applying members 18 are not equal to the lengths of handles 14, allowing for greater or lesser force or pressure at tissue-contacting surfaces 22 than is applied at finger holes 16. For example, where the lengths of pressure-applying members 18 are less than the lengths of handles 14, greater force may be applied at tissue-contacting surfaces 22 than is applied at finger holes 16. Releasable ratcheting mechanism 26 includes two complementary portions configured to engage with each other and to lock handles 14 in a closed position, maintaining pressure or force between tissue-contacting surfaces 22 while the locking mechanism 26 is engaged.

A non-invasive blood vessel-occluding system 10 also includes a sensor component 28, such as a blood flow detection system, which includes a sensor 30 and a cable 32 having a proximal connector 34 configured to operably engage with a sensor control device 36. A connector 34 is preferably a releasable connector configured to readily engage and disengage with a sensor control device 36. Alternatively, a cable 32 may directly and permanently engage a sensor control device 36 without having a connector 34. A sensor control device 36 may be configured to supply power that may be required by a sensor 30, to receive signals from a sensor 30, and to carry sensor signal outputs to a sensor controller for interpretation by an operator. A sensor 30 may be a passive sensor (e.g., configured to detect intrinsic signals indicating the presence of a blood vessel) or active (e.g., configured to emit a signal, and to detect a signal in response to, or derived from, the emitted signal). An emitted signal may be pulsed or continuous. A sensor controller 36 may produce and provide signals or signal energy used for sensing (e.g., ultrasound or infra-red signals or energy) or may provide energy to a sensor 30 to aid the sensor 30 to produce or provide signals or signal energy. Cable 32 may include an electrical cable, an optical fiber, a waveguide, other conduit for carrying energy or signals, or a combination of these.

A sensor 30 may be a blood flow sensor configured to identify and locate a blood vessel and for determining the degree of occlusion of the blood vessel. In particular, a sensor 30 may be configured to indicate the location of a blood vessel with respect to a jaw 20 of a device embodying features of the invention. A sensor 30 may thus be a blood flow sensor, but may also be a microphone (e.g., to sense heart sounds or other sounds not directly "blood flow" sounds, although turbulence due to flow may also produce detectable sounds), a pressure transducer or stress or strain gauge to detect pulsations in an artery due to heart action, a pH sensor, an electromagnetic radiation sensor, such as an infrared sensor, to detect a blood vessel (e.g., to detect hemoglobin), or other sensor. Preferably, sensor 30 is a Doppler ultrasound sensor, configured to emit and to detect ultrasound effective to detect blood flow and to locate a blood vessel.

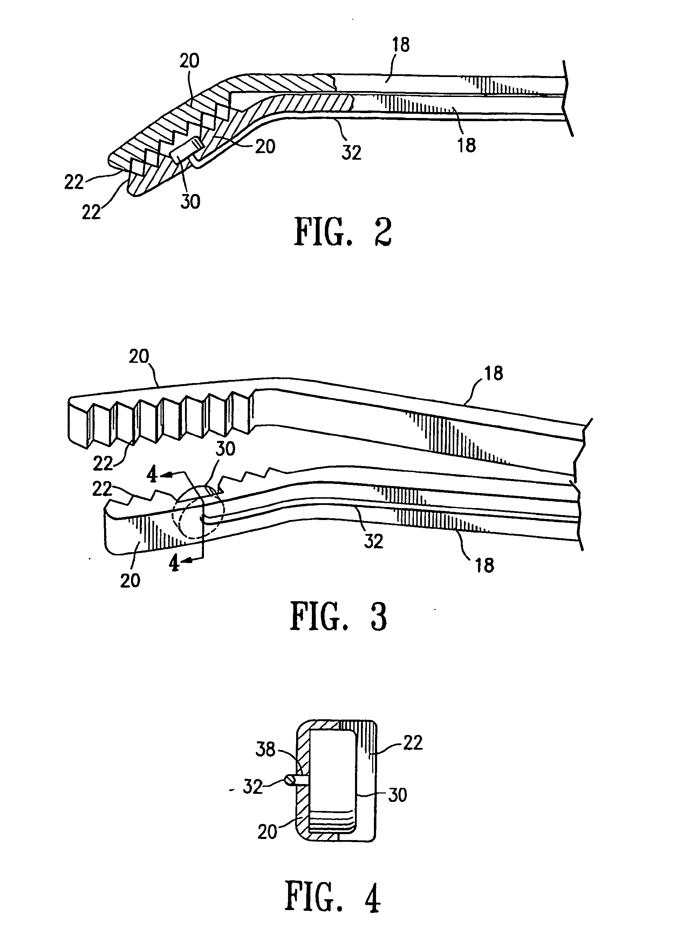

Figure 2 illustrates a distal part of a system 10 embodying features of the invention, showing portions of pressure-applying members 18, and jaws 20 having tissue-contacting surfaces 22. In the embodiment shown in this figure, jaws 20 meet pressure-applying members 18 at an angle, unlike the embodiment shown in Fig. 1 where jaws 20 meet pressure-applying members 18 to form approximately straight angles. It will be understood that jaws 20 may be disposed at any suitable angle with respect to pressure-applying members 18. A sensor 30 on one jaw 20 is also shown, with a portion of cable 32 shown disposed along a portion of a pressure-applying member 18. Tissue-contacting surfaces 22 are shown in Figure 2 disposed in close apposition to one another. Tissue-contacting surfaces 22 are placed in contact with tissue, including a portion of a blood vessel, disposed between jaws 20. Partial or complete closure of jaws 20 causes tissue-contacting surfaces 22 to apply pressure or force to the tissue effective to compress a blood vessel or the tissue around a blood vessel; the application of pressure or force is effective to compress the tissue and to occlude the blood vessel, reducing or abolishing blood flow through at least a portion of the blood vessel.

A sensor 30 may be effective to detect the location of a blood vessel and to detect blood flow in a blood vessel. Such detection may be used to direct a system 10 so as to ensure that body tissue including a portion of a blood vessel to be occluded is between jaws 20 of the clamping component 12. In preferred methods of use, the blood vessel and surrounding tissue is disposed between jaws 20 and pressure or force is applied to the tissue by tissue-contacting surfaces 22, applying pressure to the tissue, effective to compress a portion of a blood vessel and to at least partially occlude the blood vessel. Such compression and resulting occlusion of a blood vessel is effective to reduce or abolish blood flow in the vessel. Sensor 30, disposed on jaws 20, may be effective to sense the reduction or abolition of blood flow in a compressed blood vessel.

Figure 3 illustrates in greater detail the distal portion of a system 10 embodying features of the invention, showing a distal portion of a clamping component 12 having pressure-applying members 18 with jaws 20 having tissue-contacting surfaces 22. A sensor 30 is shown disposed on a jaw 20 on the tissue-contacting surface 22, with a distal portion of a cable 32 disposed opposite the tissue-contacting surface 22.

Figure 4 is a cross-sectional view of a jaw 20, taken through a sensor 30 along line 4-4 of Figure 3. The sensor 30 is connected with cable 32 by connection 38, which may be a wire, plurality of wires, optical fiber, waveguide, or other connection effective to carry signals and/or energy or power between a sensor 30, a cable 32, and a sensor controller 36. Preferably, connection 38 is a continuation of at least a portion of cable 32.

A sensor 30 may be a blood flow sensor for locating a blood vessel, and may be a passive sensor, configured to detect intrinsic signals indicating the presence of a blood vessel (i.e., a sound sensor, a motion sensor, a pH sensor, or other sensor configured to detect a physical, chemical, electrical, or physiological indication of the location of a blood vessel). In other embodiments, a blood flow sensor for locating a blood vessel may be an active sensor, configured to emit energy or a signal, and configured to detect signals in response to, or derived from, the emitted energy or signal indicating the presence of a blood vessel (i.e., a source of ultrasound having an ultrasound sensor configured to detect ultrasound reflections from a blood vessel, a source of infrared radiation configured to detect reflections from a blood vessel, or other source of energy and a sensor configured to detect a response indicating the location of a blood vessel). The operation of a sensor may be aided by an energy source (which may be provided by a sensor controller 36), which may directly provide the energy detected by the sensor, or which may aid the sensor to provide the energy to be sensed. For example, an energy source may provide electrical energy which aids an ultrasound sensor to produce and to detect ultrasound energy (as, e.g., in the MedaSonics® CardioBeat® Blood Flow Doppler with Integrated Speaker (Cooper Surgical, Inc., Trumbull CT 06611)). Other commercially available Doppler ultrasound sensors suitable for use in the present invention include the Koven model ES 100X MiniDop VRP-8 probe (St. Louis, MO) and the DWL/Neuro Scan Medical Systems' Multi-Dop B+ system (Sterling, VA).

Non-invasive blood vessel occluding devices embodying features of the invention include clamping devices having a pressure-applying member configured to apply pressure or force to a blood vessel and a blood flow sensor. A pressure-applying member may have a distal portion configured to engage tissue. Non-invasive blood vessel occluding devices embodying features of the invention may have two, or more, pressure-applying members. Two pressure-applying members maybe disposed opposite each other and configured to move and/or to apply pressure or force towards each other, such as to close together, effective to engage tissue and to clamp a blood vessel between them. Alternatively, a pressure-applying member may have two portions disposed in apposition to one another, effective to clamp tissue between the portions.

Closure of a blood vessel, which may be partial or total, is effected by pressure applied through a body wall, such as the vaginal mucosa. Sufficient pressure or force applied to tissue is effective to apply pressure to that tissue and to underlying tissues and so to compress and to at least partially occlude a blood vessel. An amount of pressure applied through a body wall to effect closure of a blood vessel may be between about 103.4 kPa (15 psi) and about 861.9 kPa (125 psi), and may preferably be between about 206.8 kPa (30 psi) and about 413.7 kPa (60 psi). For example, where the pressure-applying surface has a surface area of about 1.03 cm² (0.16 square inches) (e.g., a surface with dimensions of about 1.3 cm² (0.2 inches) by about 5.16 cm² (0.8 inches)), the amount of force applied by a non-invasive artery occluding device embodying features of the invention is preferably between about 1.36 kg (3pounds) and about 9.07 kg (20 pounds) and more preferably between about 2.72 kg (6 pounds) and about 4.08 kg (9 pounds).

A sensor for detecting or locating a blood vessel may be any sensor configured to detect a blood vessel in place within body tissue. Such a sensor may detect sound, such as heart sounds, or other sounds intrinsically associated with blood vessels. Alternatively, a sensor for locating an artery may produce or be associated with artificially created light or sound, such as ultrasound, and detect reflections or other signals derived from the artificially-produced light or sound. In preferred embodiments, a sensor may be a blood flow sensor. A blood flow sensor, such as a Doppler blood flow sensor, may be disposed perpendicular to the tissue-contacting surface 22 of a jaw 20, effective that only arteries facing a jaw 20, or within the jaws 20, are detected by the blood flow sensor.

A sensor may detect a blood vessel, or blood flow, or signals related to the location of a blood vessel or of blood flow, in a particular direction. For example, a sensor disposed on a tissue-contacting surface of a pressure-applying member, such as a jaw of a clamp, may detect signals from a direction perpendicular to the surface of the jaw, and so be effective to locate blood vessels or detect blood flow opposite the jaw. Such an orientation is effective to insure that a blood vessel to be occluded is positioned opposite a jaw, and between a pair of jaws, and so is properly placed for occlusion. A sensor may also be configured to detect signals from directions parallel to a tissue-contacting surface, or at some other angle with respect to a tissue-contacting surface; such configurations are useful, for example, for directing the movement of a non-invasive artery occluding device towards a blood vessel.

A blood flow sensor preferably includes Doppler ultrasound sensor. A blood flow sensor may be disposed on a clamping member, preferably on a distal portion configured to engage tissue, more preferably near the middle of the distal portion. A blood flow sensor disposed on a pressure-applying member may be configured to detect blood flow in a blood vessel near to the pressure-applying member, and may be configured to detect blood flow in a blood vessel clamped by a pressure-applying member or between pressure-applying members. Non-invasive blood vessel occluding devices embodying features of the invention may include more than one blood flow sensor. Preferred blood flow sensor include Doppler ultrasound blood flow sensors and near infrared blood flow sensors.

A non-invasive blood vessel occluding device embodying features of the invention may be configured to lock into a clamping position. Such a locked configuration may be temporary and releasable, or may be permanent. Non-invasive blood vessel occluding devices embodying features of the invention may have a locking mechanism, such as a ratchet, configured to hold at least one pressure-applying member in a pressure-applying position. Such locking mechanisms may include a release mechanism effective to allow the cessation of pressure or force application when desired. Thus, a non-invasive blood vessel occlusion device embodying features of the invention may be configured to release a locking mechanism effective to relieve the occlusion of a blood vessel by ending the application of pressure or force that had been previously applied to occlude a blood vessel.

The apparatus and systems of the present invention are configured for use within a body cavity and for use adjacent a patient's skin or other body surface, but are non-invasive and configured for external use. Clamping devices may be of any suitable size, which is determined in part by the location and dimension of the artery to be occluded. The handle, jaws, and if present, connecting portion, are configured to allow access to tissue adjacent a blood vessel such as a uterine artery and to provide a clamping pressure or force to the tissue sufficient to occlude the blood vessel to reduce or abolish blood flow in it.

The inventors have discovered that uterine arteries in human females are located adjacent the vaginal mucosa at a location within a few centimeters (cm), or within less than an inch to a few inches, of the vaginal fomix. Thus, for accessing and occluding a uterine artery, the dimensions of a vagina help to determine suitable sizes for clamping devices and clamp applicators embodying features of the invention so that at least a portion of a vaginal clamp is configured to fit within a vagina, and can may readily reach the vaginal fornix when operated from outside of a patient's body. For example, a clamping device may be between about 1.27 cm (0.5 inch) and about 40.6 cm (16 inches) in length, preferably between about 2.54 cm (1 inch) and about (30.5 cm) (12 inches) in length.

Apparatus and systems configured for detecting and occluding blood flow embodying features of the invention are configured to invaginate vaginal mucosa when disposed within a vagina near to a uterine artery. Such apparatus and systems are configured to invaginate vaginal mucosa without puncturing a vaginal wall; that is, without passing through the vaginal mucosa. A sensor may be configured, for example, to detect blood flow in an artery such as a uterine artery without puncturing a patient's skin or mucosal surface. A jaw or jaws of a device and of a system embodying features of the invention may be configured to compress tissue adjacent an artery such as a uterine artery without puncturing a patient's skin or mucosal surface. Thus, a vaginal clamp embodying features of the invention is effective to detect the location of an artery such as a uterine artery and to occlude it.

A vaginal clamp embodying features of the invention may have a jaw or jaws configured to engage a uterine artery or to engage tissue adjacent a uterine artery, and may have an ultrasound sensor, such as a Doppler ultrasound sensor, mounted in a jaw. A Doppler ultrasound sensor operating at ultrasound frequencies less than about 20 MHz, such as between about 5 MHz and about 19 MHz, preferably between about 6 MHz and about 10 MHz, more preferably at about 8 Hz, is suitable for detecting blood flow in an artery with apparatus embodying features of the invention. A sensor is preferably mounted to the face of the clamp jaw and oriented perpendicularly to the jaw face. For example, a blood flow sensor may be mounted between about 0.25 cm 0.1" and about 2.54 cm 1" from the distal tip of a clamp jaw, and is preferably mounted about 0.51 cm 0.2 to about 1.52 cm 0.6'', more preferably about 1.02 cm (0.4") from the distal tip of a clamp jaw. A clamp jaw may be configured to tightly engage tissue, i.e., may have a surface that is serrated, scored, roughened, coated with a rough material including sandpaper, or otherwise configured to grip tissue. For example, a clamp jaw may be serrated in order to obtain sufficient grip force to remain in position over a uterine artery when clamped onto vaginal mucosa. A non-invasive artery occluding device may have more than two jaws. Multiple jaws are preferably disposed approximately symmetrically about a central axis, and configured so that all jaws approach a central position when closed, so that, for example, three jaws may be oriented approximately 120° from each other and disposed to close to a central point effective to capture tissue between them.

The dimensions of a vaginal clamp embodying features of the invention are chosen to facilitate use within a vagina, and so that the clamp may readily reach the vaginal fornix when operated from outside of a patient's body.

A jaw or jaws may be configured to join with the connecting portion on a line substantially parallel to a line along the connecting portion, or may join at an angle to such a line. An angle between a jaw or jaws and a connecting portion may be acute or may be obtuse. In preferred embodiments, the connection between a jaw or jaws and a connecting portion or portions is a rigid connection; in some embodiments, a jaw may be an extension of a connecting portion, and both may be formed of a single piece of material.

devices embodying features of the invention may be used to occlude any artery; in the following discussion, the uterine artery is used as an example. It will be understood that the methods and devices discussed in regard to this example may also be applied to any other artery, particularly any other artery located near a body wall such as a vaginal wall, a rectal wall, and abdominal wall, skin surface, or other body surface.

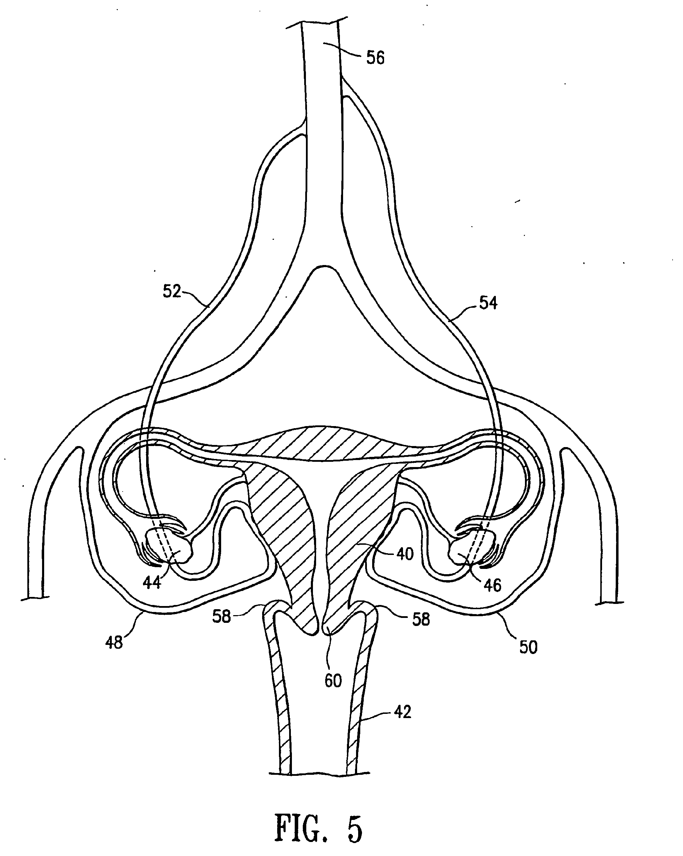

Figure 5 illustrates a typical human female reproductive system, including a uterus 40, vagina 42, right ovary 44, and left ovary 46. Blood is supplied to the uterus 40 primarily via the right uterine artery 48 and the left uterine artery 50, and secondarily via the right ovarian artery 52 and the left ovarian artery 54, all of which are supplied by the aorta 56. Note the close apposition of the uterine arteries 48 and 50 to the vaginal fornix 58 and to the uterine cervix 60.

An exemplary method of occluding an artery includes sensing an artery, and compressing an artery with a clamping device having a blood flow sensor. Sensing an artery may include sensing blood flow, such as blood flow in an artery. Compressing an artery may include grasping tissue near to an artery, and may include compressing tissue surrounding an artery effective to compress the artery.

Another exemplary method of occluding a uterine artery includes applying an artery occluding device to the artery so that blood flow through the artery is reduced, or is abolished. Such occlusion may be effected by clamping an artery such as a uterine artery. Clamping of a uterine artery may be accomplished by applying a clamping device to tissue near to a uterine artery effective to compress the uterine artery.

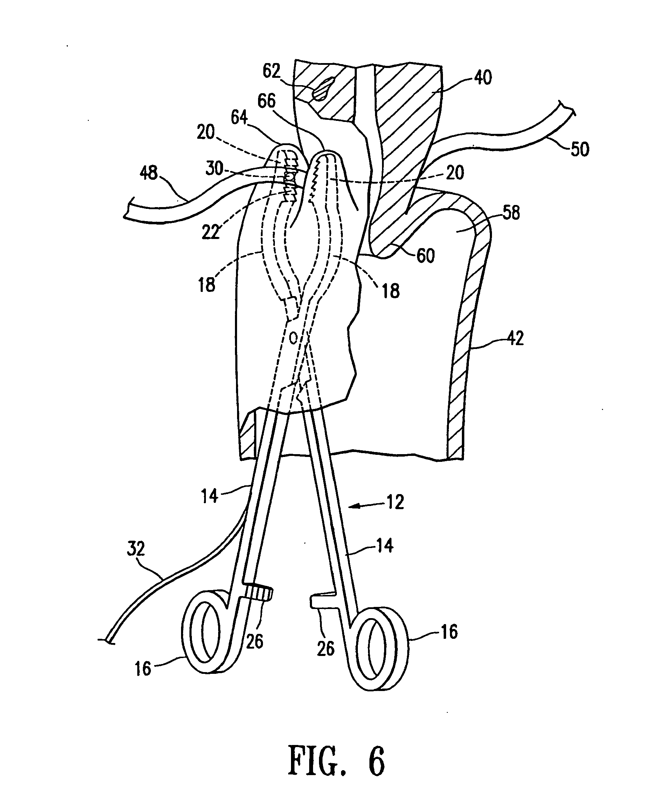

Figure 6 illustrates the use of a non-invasive artery occluding device embodying features of the invention. A vaginal clamp 12 (the clamping component of a non-invasive artery occluding system 10, only parts of which are illustrated in Figure 6) is shown partially within a vagina 42 of a female patient having a uterus 40 with a uterine fibroid 62 (one of the several medical conditions which may be treated by occlusion of the uterine arteries). The uterine arteries 48 and 50 approach the uterus 40 not far from the vaginal fornix 58 and the uterine cervix 60. The vaginal clamp 12 has handles 14 with finger holes 16, and pressure-applying members 18 with jaws 20 having tissue-contacting surfaces 22. The vaginal clamp 12 also includes a sensor 30 on a jaw 20 facing the patient's tissue, and communicating with other parts of the system 10 (not shown in the Figure) via a cable 32.

A uterine artery may be accessed via the vagina of a patient, and compressing a uterine artery may be accomplished by compressing a portion of the vaginal wall around a portion of a uterine artery. The vaginal clamp is able to access the uterine arteries via the vagina 42, by pressing with jaws 20 on the vaginal wall near the vaginal fornix 58 so as to distend portions 64 and 66 of the vaginal wall to more closely approach the right uterine artery 48. Pressure from jaws 20 is thus effective to invaginate the vaginal wall in order to bring tissue around uterine artery 48 as shown in Figure 6. Sensor 30 is effective to detect the presence of and to locate uterine artery 48, and to detect blood flow in the artery 48. Sensor 30 may be used to aid in positioning jaws 20 and tissue-contacting surfaces 22 to best surround uterine artery 48 by vaginal wall portions 64 and 66 and associated tissue. Closing jaws 20 presses tissue-contacting surfaces 22 more strongly into the vaginal wall portions 64 and 66, compressing uterine artery 48 and other tissue between the jaws 20, effective to occlude uterine artery 48. Sensor 30 may be used to detect the resulting reduction or abolition of blood flow in uterine artery 48, and to adjust the amount of pressure or force used in order to effect the desired amount of reduction in blood flow and to confirm abolition of blood flow if desired. A locking mechanism 26 may be used to maintain the desired amount of pressure or force on the tissue for a desired amount of time. Blood flow in the left uterine artery 50 may be similarly occluded, by the same vaginal clamp 12 (after release of the occlusion of the right uterine artery 48) or by a different vaginal clamp 12 (thus allowing simultaneous clamping and occlusion of both uterine arteries).

A clamping device of the invention may be a releasable clamping device, so that a uterine artery may remain occluded for only a limited time. A suitable limited time may be between about 0.2 hours and about 12 hours, or preferably between about 0.5 hours and about 4 hours.

Non-invasive artery occluding devices embodying features of the invention may be made from any suitable material or combination of materials, including metals such as stainless steel and shape memory alloys such as nickel titanium alloys, plastics, ceramics, and other materials known in the art. Biocompatible polymers, such as for example, polycarbonate, polysulfone, polyester, polyacetal, and other polymers may be particularly suitable for embodiments of the invention. The device or system may be designed for single use (disposable) or may be sterilizable and capable of multiple use.

While particular forms of the invention have been illustrated and described, it will be apparent that various modifications can be made without departing from the spirit and scope of the invention. Accordingly, it is not intended that the invention be limited to the specific embodiments illustrated. It is therefore intended that this invention be defined by the scope of the appended claims.

## Claims

1. A uterine artery occlusion device, comprising:
a. a pair of elongated clamping members (18) which have proximal and distal ends, which have opposed tissue-contacting pressure applying surfaces (22) on their distal ends, which are pivotally connected at a location (24) proximally spaced from the distal ends and distally spaced from the proximal ends and which have manually manipulative proximal portions (16) proximal to the pivotal connection (24) configured to extend out of the patient; and **characterised in that** the device has
b. at least one blood flow detecting sensor (30) at the tissue-contacting pressure applying surfaces (22) on the distal end of one of the clamping members (18) to facilitate location or monitoring of the uterine artery to be occluded.

2. The uterine artery occlusion device of claim 1, wherein the at least one blood flow sensor (30) is selected from sensors consisting of ultrasound sensors, pressure sensors, strain sensors, stress sensors, chemical sensors, electromagnetic radiation sensors, and combinations thereof.

3. The uterine artery occlusion device of claim 2, wherein said blood flow sensor (30) comprises a Doppler ultrasound sensor.

4. The uterine artery occlusion device of claim 3, wherein said Doppler ultrasound sensor is configured to sense ultrasound energy having a frequency of between about 5 MHz and about 19 MHz.

5. The uterine artery occlusion device of claim 4, wherein said Doppler ultrasound sensor is configured to sense ultrasound energy having a frequency of between about 6 MHz and about 10 MHz.

6. The uterine artery occlusion device of claim 5, wherein said Doppler ultrasound sensor is configured to sense ultrasound energy having a frequency of about 8 MHz.

7. The uterine artery occlusion device of claim 2, wherein said sensor (30) is an electromagnetic radiation sensor configured to sense electromagnetic radiation having a wavelength of between about 500 nm and about 2000 nm.

8. The uterine artery occlusion device of claim 2, wherein said sensor (30) is an electromagnetic radiation sensor configured to sense electromagnetic radiation having a wavelength of between about 700 nm and about 1000 nm.

9. The uterine artery occlusion device of claim 1, wherein at least one blood flow sensor (30) has a sensing direction perpendicular to said tissue-contacting surface (22).

10. The uterine artery occlusion device of claim 1, wherein a pressure-applying member (18) has a distal tip and wherein said sensor (30) is spaced between about 0.254 cm (0.1 inch) and about 2.54 cm (1 inch) proximal of said distal tip.

11. The uterine artery occlusion device of claim 10, wherein said sensor (30) is disposed at a location between about 0.508 cm (0.2 inch) and about 1.524 cm (0.6 inch) proximal of said distal tip.

12. The uterine artery occlusion device of claim 11, wherein said sensor (30) is disposed at a location about 1.016 cm (0.4 inch) proximal of said distal tip.

13. The uterine artery occlusion device of claim 1, wherein said non-invasive blood vessel occlusion device is configured for intravaginal use.

14. The uterine artery occlusion device of claim 1, wherein the length between the distal ends and the proximal ends of the elongated clamping members (18) is about 1.27 cm (0.5 inch) to about 40.64 cm (16 inches).

15. The uterine artery occlusion device of claim 14, wherein said length is about 2,54 cm (1 inch) to about 30.48 cm (12 inches).

16. The uterine artery occlusion device of claim 1, wherein the proximal portions (14) of the elongated clamping members are configured to be releasably secured together to retain the pressure-applying surfaces on the distal ends of the clamping members in desired positions.

17. The uterine artery occlusion device of claim 16, wherein the proximal portions (14) have releasable ratchet-type locking mechanisms (26).

18. The uterine artery occlusion device of claim 1, wherein the pair of clamping members (18) are movably configured to compress tissue disposed between the pressure applying surfaces (22) upon pivotal movement of the clamping members (18).

19. The uterine artery occlusion device of claim 1, wherein the pressure applying surfaces (22) are configured to apply between about 103.4 kPa (15 psi) and about 861.9 kPa (125 psi) of pressure to the tissue disposed between the pressure applying surfaces (22).

20. The uterine artery occlusion device of claim 19, wherein the pressure applying surfaces (22) are configured to apply between about 206.8 kPa (30 psi) and about 413.7 kPa (60 psi) of pressure to the tissue disposed between the pressure applying surfaces (22).

21. The uterine artery occlusion device of claim 1, wherein at least one blood flow sensor (30) is disposed on each pressure applying surface (22).

22. The uterine artery occlusion device of claim 1, wherein a blood flow sensor controller (36) is operatively connected to the blood flow sensor (30) and has a source of electrical power.

23. The uterine artery occlusion device of claim 22, wherein the sensor controller (36) is Doppler ultrasound controller.

24. The uterine artery occlusion device of claim 23, wherein said sensor controller (36) is configured to provide an output detectable by an operator.

## Patentansprüche

1. Gebärmutterarterien-Okklusionsvorrichtung umfassend:
a) ein Paar von länglichen Klemmelementen (18), die proximale und distale Enden aufweisen, die gegenüberliegende Gewebe-kontaktierende, Druckanlegende Oberflächen (22) an ihren distalen Enden aufweisen, die schwenkbar an einer Position (24) verbunden sind, die proximal von den distalen Enden und distal von den proximalen Enden beabstandet ist und die manuell manipulierbare proximale Abschnitte (16) proximal zu der Schwenkverbindung (24) aufweisen, die konfiguriert sind, um sich aus dem Patienten zu erstrecken; und
**dadurch gekennzeichnet, dass** die Vorrichtung umfasst:
b) mindestens einen Blutflusserfassungssensor (30) an den Gewebe-kontaktierenden, Druck-anlegenden Oberflächen (22) an dem distalen Ende eines der Klemmelemente (18), um die Lokalisierung oder Überwachung der zu okkludierenden Gebärmutterarterie zu ermöglichen:

2. Gebärmutterarterien-Okklusionsvorrichtung gemäß Anspruch 1, bei der der mindestens eine Blutflusssensor (30) aus Sensoren ausgewählt ist, die aus Ultraschallsensoren, Drucksensoren, Dehnungssensoren, Spannungssensoren, chemischen Sensoren, elektromagnetischen Strahlungssensoren und Kombinationen davon bestehen.

3. Gebärmutterarterien-Okklusionsvorrichtung gemäß Anspruch 2, bei der der Blutflusssensor (30) einen Doppler-Ultraschallsensor umfasst.

4. Gebärmutterarterien-Okklusionsvorrichtung gemäß Anspruch 3, bei der der Doppler-Ultraschallsensor konfiguriert ist, um Ultraschallenergie mit einer Frequenz zwischen etwa 5 MHz und etwa 19 MHz abzufühlen.

5. Gebärmutterarterien-Okklusionsvorrichtung gemäß Anspruch 4, bei der der Doppler-Ultraschallsensor konfiguriert ist, um Ultraschallenergie mit einer Frequenz zwischen etwa 6 MHz und etwa 10 MHz abzufühlen.

6. Gebärmutterarterien-Okklusionsvorrichtung gemäß Anspruch 5, bei der der Doppler-Ultraschallsensor konfiguriert ist, um Ultraschallenergie mit einer Frequenz von etwa 8 MHz abzufühlen.

7. Gebärmutterarterien-Okklusionsvorrichtung gemäß Anspruch 2, bei der der Sensor (30) ein elektromagnetischer Strahlungssensor ist, der konfiguriert ist, um elektromagnetische Strahlung mit einer Wellenlänge zwischen etwa 500 nm und etwa 2000 nm abzufühlen.

8. Gebärmutterarterien-Okklusionsvorrichtung gemäß Anspruch 2, bei der der Sensor (30) ein elektromagnetischer Strahlungssensor ist, der konfiguriert ist, um elektromagnetische Strahlung mit einer Wellenlänge zwischen etwa 700 nm und etwa 1000 nm abzufühlen.

9. Gebärmutterarterien-Okklusionsvorrichtung gemäß Anspruch 1, bei der der mindestens eine Blutflusssensor (30) eine Abfühlrichtung senkrecht zu der Gewebe-kontaktierenden Oberfläche (22) aufweist.

10. Gebärmutterarterien-Okklusionsvorrichtung gemäß Anspruch 1, bei der ein Druck-anlegendes Element (18) eine distale Spitze aufweist, und wobei der Sensor (30) zwischen etwa 0,254 cm (0,1 Zoll) und etwa 2,54 cm (1 Zoll) proximal von der distalen Spitze beabstandet ist.

11. Gebärmutterarterien-Okklusionsvorrichtung gemäß Anspruch 10, bei der der Sensor (30) an einer Position zwischen etwa 0,508 cm (0,2 Zoll) und etwa 1,524 cm (0,6 Zoll) proximal von der distalen Spitze angeordnet ist.

12. Gebärmutterarterien-Okklusionsvorrichtung gemäß Anspruch 11, bei der der Sensor (30) an einer Position etwa 1,016 cm (0,4 Zoll) proximal von der distalen Spitze angeordnet ist.

13. Gebärmutterarterien-Okklusionsvorrichtung gemäß Anspruch 1, bei der die nicht invasive Blutgefäss-Okklusionsvorrichtung zur intravaginalen Anwendung konfiguriert ist.

14. Gebärmutterarterien-Okklusionsvorrichtung gemäß Anspruch 1, bei der die Länge zwischen den distalen Enden und den proximalen Enden der länglichen Klemmelemente (18) etwa 1,27 cm (0,5 Zoll) bis etwa 40,64 cm (16 Zoll) beträgt.

15. Gebärmutterarterien-Okklusionsvorrichtung gemäß Anspruch 14, bei der die Länge etwa 2,54 cm (1 Zoll) bis etwa 30,48 cm (12 Zoll) beträgt.

16. Gebärmutterarterien-Okklusionsvorrichtung gemäß Anspruch 1, bei der die proximalen Abschnitte (14) der länglichen Klemmelemente konfiguriert sind, um lösbar zusammen gesichert zu sein, um die Druck-anlegenden Oberflächen an den distalen Enden der Klemmelemente in gewünschten Positionen zu halten.

17. Gebärmutterarterien-Okklusionsvorrichtung gemäß Anspruch 16, bei der die proximalen Abschnitte (14) lösbare Feststellmechanismen vom Ratschentyp (26) aufweisen.

18. Gebärmutterarterien-Okklusionsvorrichtung gemäß Anspruch 1, bei der das Paar von Klemmelementen (18) bewegbar konfiguriert ist, um Gewebe, das zwischen den Druck-anlegenden Oberflächen (22) angeordnet ist, bei einer Schwenkbewegung der Klemmelemente (18) zu komprimieren.

19. Gebärmutterarterien-Okklusionsvorrichtung gemäß Anspruch 1, bei der die Druck-anlegenden Oberflächen (22) konfiguriert sind, um einen Druck zwischen etwa 103,4 kPa (15 psi) und etwa 861,9 kPa (125 psi) an das Gewebe anzulegen, das zwischen den Druck-anlegenden Oberflächen (22) angeordnet ist.

20. Gebärmutterarterien-Okklusionsvorrichtung gemäß Anspruch 19, bei der die Druck-anlegenden Oberflächen (22) konfiguriert sind, um einen Druck zwischen etwa 206,8 kPa (30 psi) und etwa 413,7 kPa (60 psi) an das Gewebe anzulegen, das zwischen den Druck-anlegenden Oberflächen (22) angeordnet ist.

21. Gebärmutterarterien-Okklusionsvorrichtung gemäß Anspruch 1, bei der mindestens ein Blutflusssensor (30) an jeder Druck-anlegenden Oberfläche (22) angeordnet ist.

22. Gebärmutterarterien-Okklusionsvorrichtung gemäß Anspruch 1, bei der ein Blutflusssensorcontroller (36) operativ mit dem Blutflusssensor (30) verbunden ist und eine Quelle von elektrischer Energie aufweist.

23. Gebärmutterarterien-Okklusionsvorrichtung gemäß Anspruch 22, bei der der Sensorcontroller (36) ein Doppler-Utraschallcontroller ist.

24. Gebärmutterarterien-Okklusionsvorrichtung gemäß Anspruch 23, bei der der Sensorcontroller (36) konfiguriert ist, um eine durch einen Bediener erfassbare Ausgabe bereitzustellen.

## Revendications

1. Dispositif d'occlusion d'artère utérine, comprenant :
a. une paire d'éléments de pincement de forme allongée (18) qui ont des extrémités proximales et distales, qui ont des surfaces d'application de pression en contact avec les tissus et opposées (22) sur leurs extrémités distales, qui sont assemblés à pivotement en un endroit (24) distant de façon proximale des extrémités distales et distant de façon distale des extrémités proximales et qui ont des parties proximales de manipulation manuelle (16) proximales par rapport à l'assemblage pivotant (24) et conçues pour s'étendre hors du patient ; et **caractérisé en ce que** le dispositif comporte :
b. au moins un capteur de détection d'écoulement de sang (30) dans les surfaces d'application de pression en contact avec les tissus (22) sur l'extrémité distale de l'un des éléments de pincement (18) pour faciliter la localisation ou la surveillance de l'artère utérine à occlure.

2. Dispositif d'occlusion d'artère utérine selon la revendication 1, dans lequel ledit au moins un capteur d'écoulement de sang (30) est choisi dans les capteurs comprenant les capteurs à ultrasons, les capteurs de pression, les capteurs de déformation, les capteurs de contrainte, les capteurs chimiques, les capteurs de rayonnement électromagnétique et leurs associations.

3. Dispositif d'occlusion d'artère utérine selon la revendication 2, dans lequel ledit capteur d'écoulement de sang (30) comprend un capteur à ultrasons à effet Doppler.

4. Dispositif d'occlusion d'artère utérine selon la revendication 3, dans lequel ledit capteur à ultrasons à effet Doppler est configuré pour mesurer une énergie ultrasonore ayant une fréquence comprise entre environ 5 MHz et environ 19 MHz.

5. Dispositif d'occlusion d'artère utérine selon la revendication 4, dans lequel ledit capteur à ultrasons à effet Doppler est configuré pour mesurer une énergie ultrasonore ayant une fréquence comprise entre environ 6 MHz et environ 10 MHz.

6. Dispositif d'occlusion d'artère utérine selon la revendication 5, dans lequel ledit capteur à ultrasons à effet Doppler est configuré pour mesurer une énergie ultrasonore ayant une fréquence d'environ 8 MHz.

7. Dispositif d'occlusion d'artère utérine selon la revendication 2, dans lequel ledit capteur (30) est un capteur de rayonnement électromagnétique configuré pour mesurer un rayonnement électromagnétique ayant une longueur d'onde comprise entre environ 500 nm et environ 2 000 nm.

8. Dispositif d'occlusion d'artère utérine selon la revendication 2, dans lequel ledit capteur (30) est un capteur de rayonnement électromagnétique configuré pour mesurer un rayonnement électromagnétique ayant une longueur d'onde comprise entre environ 700 nm et environ 1 000 nm.

9. Dispositif d'occlusion d'artère utérine selon la revendication 1, dans lequel au moins un capteur d'écoulement de sang (30) a une direction de mesure perpendiculaire à ladite surface en contact avec les tissus (22).

10. Dispositif d'occlusion d'artère utérine selon la revendication 1, dans lequel un élément d'application de pression (18) a un bout distal et dans lequel ledit capteur (30) est à une distance comprise entre environ 0,254 cm (0,1 pouce) et environ 2,54 cm (1 pouce) dudit bout distal, de façon proximale.

11. Dispositif d'occlusion d'artère utérine selon la revendication 10, dans lequel ledit capteur (30) est placé à un endroit situé entre environ 0,508 cm (0,2 pouce) et environ 1,524 cm (0,6 pouce) dudit bout distal, de façon proximale.

12. Dispositif d'occlusion d'artère utérine selon la revendication 11, dans lequel ledit capteur (30) est placé en un endroit situé à environ 1,016 cm (0,4 pouce) dudit bout distal, de façon proximale.

13. Dispositif d'occlusion d'artère utérine selon la revendication 1, dans lequel ledit dispositif d'occlusion de vaisseau sanguin non effractif est conçu pour une utilisation intra-vaginale.

14. Dispositif d'occlusion d'artère utérine selon la revendication 1, dans lequel la longueur entre les extrémités distales et les extrémités proximales des éléments de pincement de forme allongée (18) vaut d'environ 1,27 cm (0,5 pouce) à environ 40,64 cm (16 pouces).

15. Dispositif d'occlusion d'artère utérine selon la revendication 14, dans lequel ladite longueur vaut d'environ 2,54 cm (1 pouce) à environ 30,48 cm (12 pouces).

16. Dispositif d'occlusion d'artère utérine selon la revendication 1, dans lequel les parties proximales (14) des éléments de pincement de forme allongée sont conçues pour être fixées l'une à l'autre de manière amovible pour retenir les surfaces d'application de pression des extrémités distales des éléments de pincement dans des positions voulues.

17. Dispositif d'occlusion d'artère utérine selon la revendication 16, dans lequel les parties proximales (14) comportent des mécanismes de blocage déverrouillables du type à cliquet (26).

18. Dispositif d'occlusion d'artère utérine selon la revendication 16, dans lequel les éléments de pincement (18) sont configurés de manière mobile pour comprimer le tissu placé entre les surfaces d'application de pression (22) lors du mouvement pivotant des éléments de pincement (18).

19. Dispositif d'occlusion d'artère utérine selon la revendication 1, dans lequel les surfaces d'application de pression (22) sont conçues pour appliquer une pression comprise entre environ 103,4 kPa (15 psi) et environ 861,9 kPa (125 psi) au tissu placé entre les surfaces d'application de pression (22).

20. Dispositif d'occlusion d'artère utérine selon la revendication 19, dans lequel les surfaces d'application de pression (22) sont conçues pour appliquer une pression comprise entre environ 206,8 kPa (30 psi) et environ 413,7 kPa (60 psi) au tissu placé entre les surfaces d'application de pression (22).

21. Dispositif d'occlusion d'artère utérine selon la revendication 1, dans lequel au moins un capteur d'écoulement de sang (30) est placé sur chaque surface d'application de pression (22).

22. Dispositif d'occlusion d'artère utérine selon la revendication 1, dans lequel un contrôleur de capteur d'écoulement de sang (36) est connecté de façon fonctionnelle au capteur d'écoulement de sang (30) et possède une source d'alimentation électrique.

23. Dispositif d'occlusion d'artère utérine selon la revendication 22, dans lequel le contrôleur de capteur (36) est un contrôleur à ultrasons à effet Doppler.

24. Dispositif d'occlusion d'artère utérine selon la revendication 23, dans lequel ledit contrôleur de capteur (36) est configuré pour fournir une sortie détectable par un opérateur.
